# EUROPEAN PATENT APPLICATION

(11) **EP 2 141 160 A1**
(43) Date of publication of application: **06.01.2010**
(21) Application number: 08722426.7
(22) Date of filing: 19.03.2008
(51) Int. Cl.: C07D 405/14, A61K 9/14, A61K 9/20, A61K 31/4178, A61P 9/00, A61P 9/12, A61P 43/00

(54) **GROUND CRYSTAL OF OLMESARTAN MEDOXOMIL**

(30) Priority: 23.03.2007 JP 2007075701
(71) Applicant: Daiichi Sankyo Company, Limited, Chuo-ku Tokyo 103-8426 (JP)
(72) Inventor: HASEGAWA, Susumu, Hiratsuka-shi Kanagawa 254-0014 (JP); HAMAURA, Takeshi, Hiratsuka-shi Kanagawa 254-0014 (JP)
(74) Representative: Andrews, Timothy Stephen
(86) International application number: PCT/JP2008/055036
(87) International publication number: WO 2008/117707

(57) **Abstract**

The present invention provides a medicament containing olmesartan medoxomil wherein the dissolution property from the medicament is controlled. An olmesartan medoxomil having a particle diameter at 90% cumulative volume of 75 µm or less, a process for the production of a medicament containing olmesartan medoxomil which uses the olmesartan medoxomil having such particle diameter, and the like are also provided.

## Description

### [TECHNICAL FIELD]

The present invention relates to olmesartan medoxomil having a particular particle diameter, a medicament containing olmesartan medoxomil the dissolution of which property is controlled, and to a production process thereof.

### [BACKGROUND ART]

Olmesartan medoxomil ((5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate) is known to be an excellent angiotensin II receptor blocker, and is useful as a treatment drug or a prevention drug of hypertension, heart disease and the like (refer to Patent Document 1).

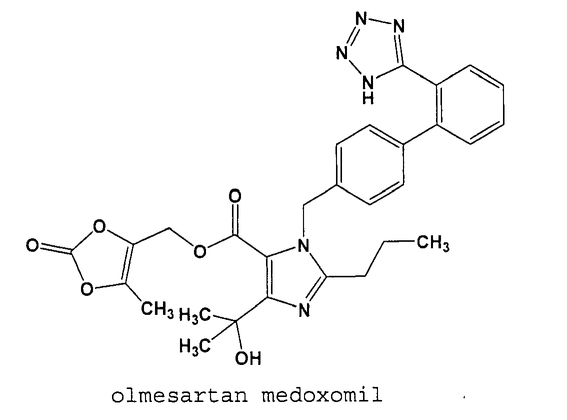

Here, although the physicochemical properties of olmesartan medoxomil are disclosed in non-Patent Document 1, it is not known as to what particle diameters olmesartan medoxomil crystals have.

When producing a medicament containing olmesartan medoxomil as a pharmaceutical drug, the pharmaceutical management authority requires that bioavailability of olmesartan medoxomil concerning each lot of the medicament is within a constant range from the standard value. Since the bioavailability is correlated with the dissolution properties from a medicament, it is important to control the dissolution properties from the medicament.

In general, the dissolution properties of a drug can be improved by pulverizing the drug substance of a pharmaceutical compound so as to have a small particle diameter; however, with respect to the medicament containing olmesartan medoxomil, it was extremely difficult to predict what particle diameter is required for the crystals, in order to control the dissolution property.
[Patent Document 1] US Patent No. 5,616,599
[Non-Patent Document 1] Sankyo Research Institute Annual Report, Voi. 55, pp. 12-15, 2003

### [DISCLOSURE OF THE INVENTION]

### [PROBLEMS TO BE SOLVED BY THE INVENTION]

The object of the present invention is to provide olmesartan medoxomil having a particular particle diameter, a medicament containing olmesartan medoxomil the dissolution property of which is controlled, and a production process thereof.

### [MEANS FOR SOLVING THE PROBLEMS]

As a result of conducting extensive studies on formulation techniques which would improve the dissolution property of olmesartan medoxomil, the inventors of the present invention found that a medicament whose dissolution property is controlled can be produced by using olmesartan medoxomil having a particle diameter at 90% cumulative volume of 75 µm or less, thereby leading to completion of the present invention.

The present invention provides olmesartan medoxomil having a particle diameter at 90% cumulative volume of 75 µm or less, a process for the production of olmesartan medoxomil having such a particle diameter, a process for the production of a medicament containing olmesartan medoxomil which uses the olmesartan medoxomil having such a particle diameter, and a medicament containing olmesartan medoxomil which uses the olmesartan medoxomil having such a particle diameter.

That is, the present invention provides
(1) an olmesartan medoxomil having a particle diameter at 90% cumulative volume of 75 µm or less,
(2) an olmesartan medoxomil having a particle diameter at 90% cumulative volume of 66 µm or less,
(3) an olmesartan medoxomil having a particle diameter at 90% cumulative volume of 57 µm or less,
(4) an olmesartan medoxomil having a particle diameter at 90% cumulative volume of 75 µm or less and a particle diameter at 99% cumulative volume of 156 µm or less,
(5) an olmesartan medoxomil having a particle diameter at 90% cumulative volume of 66 µm or less and a particle diameter at 99% cumulative volume of 137 µm or less,
(6) an olmesartan medoxomil having a particle diameter at 90% cumulative volume of 57 µm or less and a particle diameter at 99% cumulative volume of 119 µm or less,
(7) a process for the production of the olmesartan medoxomil as described in (1) through (6),
(8) a process for the production of a medicament containing olmesartan medoxomil which uses the olmesartan medoxomil as described in (1) through (6), and
(9) a medicament containing olmesartan medoxomil and the like which is produced by using the olmesartan medoxomil as described in (1) through (6).

### [EFFECT OF THE INVENTION]

According to the present invention, a medicament containing olmesartan medoxomil, the dissolution property of which is controlled, and a production process thereof can be provided.

### [BEST MODE FOR CARRYING OUT THE INVENTION]

Olmesartan modoxomil, which is an active ingredient of the present invention, can be produced easily in accordance with the method described in US Patent No. 5,616,599 and the like.

As for the process for the production of the medicament containing olmesartan medoxomil according to the present invention, procedures are not particularly limited so long as it uses "olmesartan medoxomil having a particle diameter at 90% cumulative volume of 75 µm or less", and the medicament can be produced by using ordinary methods described in publications such as Powder Technology and Pharmaceutical Processes (D. Chulia et al., Elsevier Science Pub Co (December 1, 1993)).

The "olmesartan medoxomil having a particle diameter at 90% cumulative volume of 75 µm or less" can be produced by pulverizing crystals having a larger particle diameter.

As for methods to pulverize crystals, knife type, hammer type, pin type, jet type and the like can be mentioned.

The knife type is a method which pulverizes with impact generated by rotating a blade. The typical apparatus is Power Mill (Fuji Paudal Co., Ltd.).

The hammer type is a method which pulverizes with impact generated by rotating a hammer. The typical apparatuses are Atomizer (Fuji Paudal Co., Ltd.), Pulverizor (Hosokawa Micron Corporation) and the like.

The pin type is a method which pulverizes with impact generated by rotating a pin disc having a plurality of pins at high speed. The typical apparatuses are Fine Compact Mill (Hosokawa Micron Corporation) and the like.

The jet type is a method which pulverizes by allowing a material to be swept by a high speed airstream of compressed air, and by allowing the particles to collide with each other or with a pulverizing unit. Typical apparatuses are Jet Mill (Nippon Pneumatic MFG. Co., Ltd.), Counter Jet Mill (Hosokawa Micron Corporation) and the like.

The particle diameter of olmesartan medoxomil of the present invention is not limited so long as it is a size which can improve the dissolution property of the active ingredient; however, the particle diameter at 90% cumulative volume is preferably 75 µm or less, more preferably 66 µm or less, even more preferably 57 µm or less, and the particle diameter at 99% cumulative volume is preferably 156 µm or less, more preferably 137 µm or less, and even more preferably 119 µm or less.

The medicament of the present invention can, if necessary, contain an additive such as a suitable pharmacologically acceptable excipient, lubricant, binder, disintegrant, emulsifier, stabilizer, corrective or diluent.

As for the "excipients" used, organic excipients including sugar derivatives such as lactose, sucrose, glucose, mannitol or sorbitol; starch derivatives such as corn starch, potato starch, α-starch or dextrin; cellulose derivatives such as crystalline cellulose; gum Arabic; dextran; and pullulan; and inorganic excipients including silicate derivatives such as light anhydrous silicic acid, synthetic aluminum silicate, calcium silicate or magnesium metasilicate aluminate; phosphates such as dibasic calcium hydrogenphosphate; carbonates such as calcium carbonate; and sulfates such as calcium sulfate can be mentioned.

As for the "lubricants" used, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium benzoate; D,L-leucine; lauryl sulfates such as sodium lauryl sulfate or magnesium lauryl sulfate; silicates such as silicic anhydride or silicate hydrate; and the aforementioned starch derivatives can be mentioned.

As for the "binders" used, hydroxypropyl cellulose, hydroxypropyl methyl cellulose, polyvinylpyrrolidone, macrogol and compounds similar to the aforementioned excipients can be mentioned.

As for the "disintegrants" used, cellulose derivatives such as low-substituted hydroxypropyl cellulose, carboxymethyl cellulose, calcium carboxymethyl cellulose or internally crosslinked sodium carboxymethyl cellulose; cross-linked polyvinylpyrrolidone; and chemically modified starches/celluloses such as carboxymethyl starch or sodium carboxymethyl starch can be mentioned.

As for the "emulsifiers" used, colloidal clays such as bentonite or bee gum; metal hydroxides such as magnesium hydroxide or aluminum hydroxide; anionic surfactants such as sodium lauryl sulfate or calcium stearate; cationic surfactants such as benzalkonium chloride; and nonionic surfactants such as polyoxyethylene alkyl ethers, polyoxyethylene sorbitan fatty acid esters or sucrose fatty acid esters can be mentioned.

As for the "stabilizers" used, para-hydroxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; and sorbic acid can be mentioned.

As for the "correctives" used, sweeteners such as sodium saccharin or aspartame; sour flavourings such as citric acid, malic acid or tartaric acid; and fragrances such as menthol, lemon or orange fragrance can be mentioned.

As for the "diluents" used, lactose, mannitol, glucose, sucrose, calcium sulfate, calcium phosphate, hydroxypropyl cellulose, microcrystalline cellulose, water, ethanol, polyethylene glycol, propylene glycol, glycerol, starch, polyvinylpyrrolidone, magnesium metasilicate aluminate, and mixtures thereof can be mentioned.

The present invention is **characterized in that** by the adoption of "olmesartan medoxomil having a particle diameter at 90% cumulative volume of 75 µm or less", the dissolution property of the active ingredient can be optimized without the use of a "solubilizing agent". However, the medicament of the present invention may also contain a "solubilizing agent", and thus a medicament with further optimized eluting property can be produced.

As for the "solubilizing agent" used, a water-soluble polymer, a surfactant and the like can be mentioned.

As for the "water-solube polymer" used, cellulose derivatives such as hydroxypropyl methyl cellulose, methyl cellulose, hydroxypropyl cellulose and sodium carboxymethyl cellulose; synthetic polymers such as polyvinylpyrrolidone, aminoalkyl methacrylate copolymers, carboxyvinyl polymers, polyvinyl alcohol and macrogol; HA "Sankyo", gum Arabic, agar, gelatin and sodium alginate can be mentioned.

As for the "surfactant" used, sodium lauryl sulfate, polysorbate and the like can be mentioned.

As for the "medicament" of the present invention, tablets (including sublingual tablets and tablets that disintegrate in the mouth), capsules (including soft capsules and microcapsules) granules, fine granules, powders, pills and troches can be mentioned for example, and is preferably powders, fine granules, granules, capsules or tablets, and most preferably tablets.

The tablets of the present invention can be obtained by granulating a base component with an excipient, binder and the like, drying and regulating the particle size, mixing the resulting granules with a lubricant and the like, and then forming them into tablets, which is a method known per se. Here, granulation can be conducted by either one of wet granulation, dry granulation or heat granulation, and in particular, it is conducted by using a high speed mixing granulator, fluid bed dryer, extrusion granulator or roller compactor for example. In addition, after granulation, operations such as drying and regulating the granules may be conducted if necessary. A mixture of base component, excipient, binder, lubricant and the like can also be directly formed into tablets.

Here, granulation refers to an operation which makes granules having a nearly uniform shape and size from raw materials which are in a form such as powders, lumps, solutions or molten liquids. Granulation in accordance with the present invention includes procedures which provide finished products such as granules, powders and fine granules, and procedures in which intermediate granular products are produced for subsequent use in the manufacture of tablets, capsules and the like.

The granulated products thus obtained can be regulated to its desired particle size, and can be formed into a medicament in the form of powders, fine granules or granules. These medicaments may also be made as capsules by filling them into capsules; otherwise they may be further added with a disintegrant, lubricant and the like if necessary and be made into a medicament in the form of tablets by compression molding by a tableting machine. Operations of mixing and granulation are both used widely in the field of medicament techniques, and those skilled in the art can appropriately carry them out. In addition, at least one layer of a film coating may be provided to the tablets.

Coating is, for example, conducted by using a film coating machine. As film coating agents, sugar coating agents, water soluble film coating agents, enteric film coating agents and sustained release film coating agents can be mentioned for example.

As for the "sugar coating agents" used, saccharose is used, and it can be used in combination with one or more additives such as talc, precipitated calcium carbonate, calcium phosphate, calcium sulfate, gelatin, gum Arabic, polyvinylpyrrolidone and pullulan.

As for the "hydrophilic film coating agents" used, cellulose derivatives such as hydroxypropyl cellulose, hydroxypropyl methyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymers and polyvinylpyrrolidone; and polysaccharides such as pullulan can be mentioned.

As for the "enteric film coating agents" used, cellulose derivatives such as hydroxypropyl methyl cellulose phthalate, hydroxypropyl methyl cellulose acetate succinate, carboxymethyl ethyl cellulose and cellulose acetate phthalate; acrylic acid derivatives such as methacrylic acid copolymer L, methacrylic acid copolymer LD and methacrylic acid copolymer S; and natural substances such as shellac can be mentioned.

As for the "sutained release film coating agents" used, cellulose derivatives such as ethyl cellulose; and acrylic acid derivatives such as aminoalkyl methacrylate copolymer RS, ethyl acrylate-methyl methacrylate copolymer emulsion can be mentioned.

As to the above coating agents, a mixture of two or more different coating agents such may also be used at a suitable ratio. In addition, the coating films may also contain suitable pharmacologically acceptable additives such as plasticizers, excipients, lubricants, opacifying agents, colorants or antiseptics, if necessary.

The dissolution property of the pharmaceutical composition of the present invention is measured in accordance with Method 2 of the Dissolution Test (Paddle Method) described in the 14th Revised Edition of the Japanese Pharmacopoeia. For example, at 50 paddle revolutions per-minute using 900 mL of Japanese Pharmacopoeia Solution 2 (JP-2, pH 6.8) for the test solution, the test solution was sampled 30 minutes after the start of testing, the sampled test solution was filtered through a membrane filter having a pore diameter of 0.45 µm, and the filtrate was subjected to quantitative analysis using a spectral photometer, thus enabling calculation of the dissolution ratio of olmesartan medoxomil.

The dosage amount of olmesartan medoxomil, which is an active ingredient in the pharmaceutical composition of the present invention, can be changed depending on various factors such as activity of each of the drugs or symptoms, age, and body weight of a patient. Although its dosage amount varies depending on the symptom, age and the like, the dose of olmesartan medoxomil in the case of oral administration is 5 mg to 80 mg, (preferably 10 mg to 40 mg), and is administered 1 to 6 times a day (preferably once a day), with respect to daily dosage to an adult human.

The pharmaceutical composition of the present invention is effective for prevention or treatment of hypertension or diseases caused by hypertension (more specifically, hypertension, heart diseases [angina pectoris, myocardial infarction, arrhythmia, cardiac insufficiency or cardiac hypertrophy], kidney diseases [diabetic nephropathy, glomerular nephritis or nephrosclerosis], or cerebrovascular disease [cerebral infarction or cerebral hemorrhage] and the like.

The present invention will be described in more detail by way of Examples and the like; however, the present invention is not intended to be limited to these.

### (Example)

### (1) Milling procedure (obtainment of pulverized crystals)

Fine Impact Mill (Hosokawa Micron Corporation) was used, and the rotation speed (8,000-18,000 rpm) and feeding speed of the drug substance (200-750 g/min) were varied to obtain milled products having various particle diameters.

### (2) Medicament forming procedure (production of olmesartan medoxomil 20 mg tablets)

The pulverized product of olmesartan medoxomil obtained in Example (1) (12.5%), lactose (66.3%), L-HPC (12.5%), HPC-L (1.9%) were loaded into a high speed agitation granulator (Vertical Granulator (Powrex Corporation)) and kneading was conducted for a particular period of time by using purified water. The kneaded product was sieved by Fiore Mini (Tokuju Co., Ltd.), dried by a fluid bed dryer (Flow Coater (Freund Corporation)), and was screened by Fiore Mini. The granules obtained were added with Avicel (Registered Trademark) (6.3%) and magnesium stearate (0.6%), mixed by a V Shaped Mixer (Tokuju Co., Ltd.), and then tablets were made by a tableting machine (Cleanpress Correct 24 or Vigro 0524SSIAX (both of them manufactured by Kikusui Seisakusho Ltd.)).

### (3) Particle diameter measurement of milled product

The particle diameter of the milled olmesartan medoxomil obtained in Example (1) was measured under the following conditions.
Apparatus: HELOS&RODOS dry powder particle size analyzer
Lens: R3
Dispersive pressure: 1.5 bar
Trigger condition: 2s-1000ms-k15-0.5%
Feeder: VIBRI (feeding: 70%)

### (4) Measurement of dissolution ratio of pulverized olmesartan medoxomil

According to the Method 2 of the Dissolution Test (Paddle Method) described in the 14th Revised Edition of the Japanese Pharmacopoeia, at 200 paddle revolutions per-minute using 900 mL of Japanese Pharmacopoeia Solution 2 (JP-2) for the test solution, 20 mg of milled olmesartan medoxomil prepared per 1 Bessel was added. The test solution was sampled at 30 minutes after the start of the testing, the sampled test solution was filtered through a membrane filter having a pore diameter of 0.45 µm, and the dissolution ratio of olmesartan medoximil was measured by spectrophotometer. Calculation of the dissolution ratio was conducted based on "absorbance of filtrate at 257 nm - absorbance of filtrate at 338 nm".

### (5) Measurement of dissolution ratio of olmesartan medoxomil 20 mg tablet

According to the Method 2 of the Dissolution Test (Paddle Method) described in the 14th Revised Edition of the Japanese Pharmacopoeia, at the paddle revolution of 50 revolutions per minute and using 900 mL of Japanese Pharmacopoeia Solution 2 (JP-2) for the test solution, 1 tablet of olmesartan medoxomil 20 mg tablet per 1 Bessel was added. Test solution was sampled at 30 minutes after the start of testing, the sampled test solution was filtered through a membrane filter having a pore diameter of 0.45 µm, and the dissolution ratio of olmesartan medoximil was measured by spectrophotometer. Calculation of the dissolution ratio was conducted based on "absorbance of filtrate at 257 nm - absorbance of filtrate at 338 nm".

Positive correlation was observed between the dissolution ratio of the pulverized crystals and that of the medicament. In addition, negative correlation was observed between the particle diameter at 90% cumulative volume of the pulverized crystals and the dissolution ratio of the pulverized crystals.

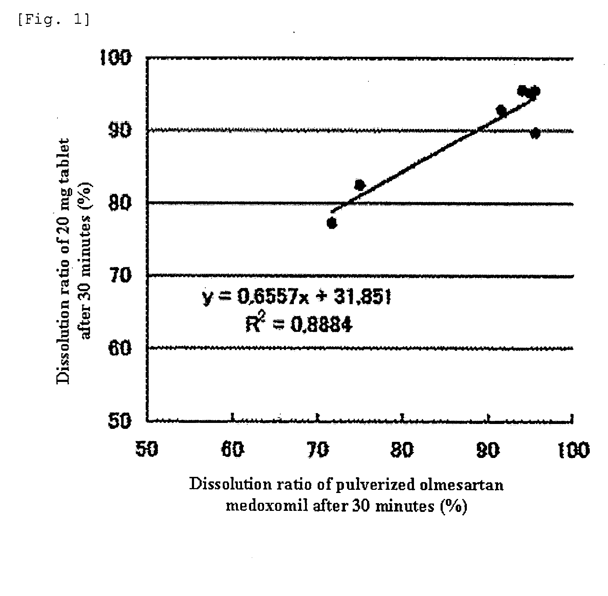

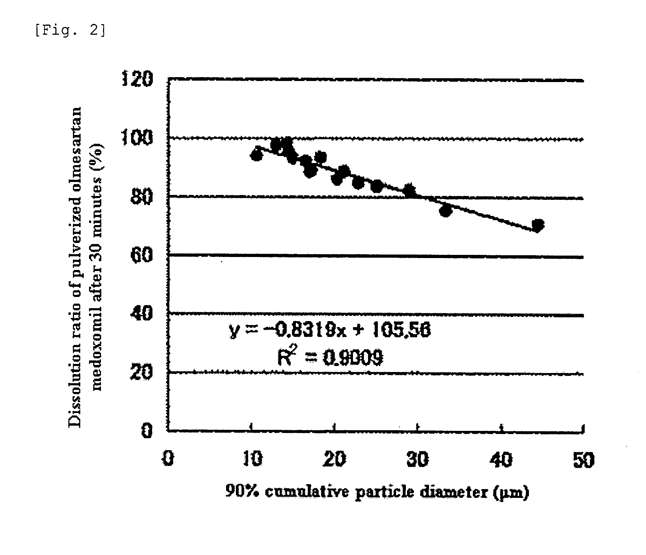

When the dissolution ratio of the drug substance is calculated from the regression equation in Fig. 1 by setting the desired dissolution ratio of the medicament to 60%, it is 42.93%. Here, when the particle diameter that satisfies this dissolution ratio is calculated from the regression equation in Fig. 2, the particle diameter at 90% cumulative volume is 75.3 µm. In addition, when the dissolution ratio of the drug substance is calculated from the regression equation in Fig. 1 by setting the desired dissolution ratio of the medicament to 65%, it is 50.56%. Here, when the particle diameter that satisfies this dissolution ratio is calculated from the regression equation in Fig. 2, the particle diameter at 90% cumulative volume is 66.1 µm. Further, when the dissolution ratio of the drug substance is calculated from the regression equation in Fig. 1 by setting the desired dissolution ratio of the medicament to 70%, it is 58.18%. Here, when the particle diameter that satisfies this dissolution ratio is calculated from the regression equation in Fig. 2, the particle diameter at 90% cumulative volume is 57.0 µm.

Accordingly, with respect to a medicament of olmesartan medoxomil, when a medicament which elutes 60% or more is prepared, the particle diameter at 90% cumulative volume of olmesartan medoxomil is required to be 75 µm or less. In addition, when a medicament which elutes 65% or more is prepared, the particle diameter at 90% cumulative volume of olmesartan medoxomil is required to be 66 µm or less. Further, when a medicament which elutes 70% or more is prepared, the particle diameter at 90% cumulative volume of olmesartan medoxomil is required to be 57 µm or less.

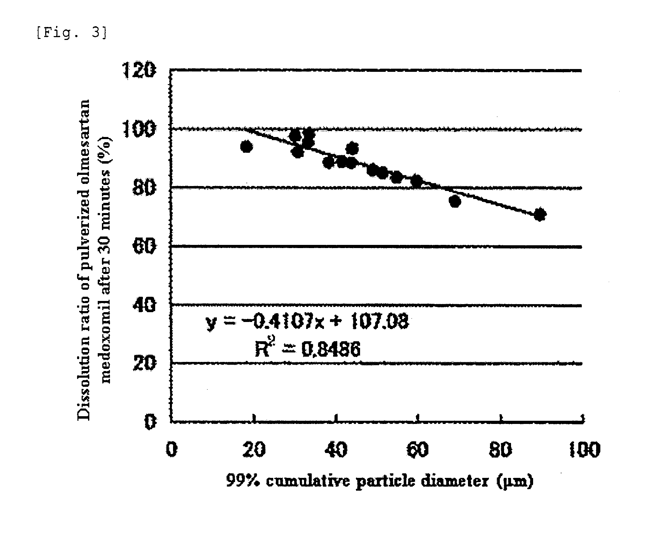

When evaluation is made of the particle diameter at 99% cumulative volume in a similar manner by using Fig. 3, with respect to a medicament of olmesartan medoxomil, when a medicament which elutes 60% or more is prepared, the particle diameter at 99% cumulative volume of olmesartan medoxomil is required to be 156 µm or less. In addition, when a medicament which elutes 65% or more is prepared, the particle diameter at 99% cumulative volume of olmesartan medoxomil is required to be 137 µm or less. Further, when a medicament which elutes 70% or more is prepared, the particle diameter at 99% cumulative volume of olmesartan medoxomil is required to be 119 µm or less.

### [INDUSTRIAL APPLICABILITY]

According to the present invention, a medicament containing olmesartan medoxomil wherein the dissolution property from a drug is controlled, can be obtained.

### [BRIEF DESCRIPTION OF THE DRAWINGS]

[Fig. 1] Correlation between dissolution ratio of pulverized olmesartan medoxomil after 30 minutes and dissolution ratio of olmesartan medoxomil 20 mg tablet after 30 minutes
[Fig. 2] Correlation between dissolution ratio of pulverized olmesartan medoxomil after 30 minutes and particle diameter at 90% cumulative volume
[Fig. 3] Correlation between dissolution ratio of pulverized olmesartan medoxomil after 30 minutes and particle diameter at 99% cumulative volume

## Claims

1. An olmesartan medoxomil having a particle diameter at 90% cumulative volume of 75 µm or less.

2. An olmesartan medoxomil having a particle diameter at 90% cumulative volume of 66 µm or less.

3. An olmesartan medoxomil having a particle diameter at 90% cumulative volume of 57 µm or less.

4. An olmesartan medoxomil having a particle diameter at 90% cumulative volume of 75 µm or less and a particle diameter at 99% cumulative volume of 156 µm or less.

5. An olmesartan medoxomil having a particle diameter at 90% cumulative volume of 66 µm or less and a particle diameter at 99% cumulative volume of 137 µm or less.

6. An olmesartan medoxomil having a particle diameter at 90% cumulative volume of 57 µm or less and a particle diameter at 99% cumulative volume of 119 µm or less.

7. A process for the production of the olmesartan medoxomil according to Claims 1 to 6.

8. A process for the production of a medicament containing olmesartan medoxomil which uses the olmesartan medoxomil according to Claims 1 to 6.

9. A medicament containing olmesartan medoxomil which is produced by using the olmesartan medoxomil according to Claims 1 to 6.
